**Europäisches Patentamt**

**European Patent Office**

**Office européen des brevets**

(11) Veröffentlichungsnummer: **0 089 417**
**B1**

# EUROPÄISCHE PATENTSCHRIFT

(12)

(45) Veröffentlichungstag der Patentschrift:
26.02.86

(21) Anmeldenummer: **82111957.5**

(22) Anmeldetag: **23.12.82**

(51) Int. Cl.⁴: **C 07 D 307/88**

(54) **Verfahren zur Herstellung von Phthalid.**

(30) Priorität: **18.01.82  DE 3201300**

(43) Veröffentlichungstag der Anmeldung:
**28.09.83 Patentblatt 83/39**

(45) Bekanntmachung des Hinweises auf die Patenterteilung:
**26.02.86 Patentblatt 86/9**

(84) Benannte Vertragsstaaten:
**BE CH DE FR GB IT LI NL**

(56) Entgegenhaltungen:
**DE - A - 2 250 066**
**US - A - 2 079 325**
**US - A - 2 114 696**

(73) Patentinhaber: **DYNAMIT NOBEL
AKTIENGESELLSCHAFT, Postfach 1209,
D-5210 Troisdorf, Bez. Köln (DE)**

(72) Erfinder: **aus der Fünten, Helmut, Dr., Buttergasse 1,
D-5216 Niederkassel-Mondorf (DE)**
Erfinder: **Vogt, Wilhelm, Dr., Kleiststrasse 39,
D-5000 Köln 40 (DE)**

**Beschreibung**

Gegenstand der vorliegenden Erfindunge ist ein Verfahren zur Herstellung von Phthalid durch katalytische Hydrierung von Phthalsäureanhydrid mit Hilfe von einem auf einem Trägermaterial fixierten Nickelkatalysetor.

Die katalytische Hydrierung von Phthalsäureanhydrid in Gegenwart von Nickel- oder Platinkatalysatoren in verschiedenen Lösungsmitteln ist bekannt (vgl. Houben-Weyl "Methoden der organischen Chemie", 6/2, (1963), Seiten 732 bis 733). Die Ausbeuten bei diesen bekannten Verfahren liegen je nach eingesetztem Lösungsmittel und verwendeten Katalysatoren maximal bei 75 %. So erhält man z.B. bei Verwendung von Raney-Nickel in absolutem Ethanol bei einem $H_2$-Druck von 165 bar bei 160 °C Reaktionstemperatur Phthalidausbeuten von 73 %; beim Einsatz von Essigsäurehutylester als Lösungsmittel liegt die Ausbeute bei etwa 71 %, wenn die Reduktion bei 160 °C und einem Druck von 130 bar durchgeführt wird (US-PS 2,114,696-Beispiel 1). Diese Ausbeuten sind für technische Verfahren ungenügend und auch nur bei Einsatz von relativ reinem Phthalsäureanhydrid erzielbar. Weiterhin nachteilig wirkt sich bei diesem Verfahren aus, daß bei einem relativ hohen Wasserstoffdruck gearbeitet werden muß.

Es bestand deshalb die Aufgabe, die katalytische Hydrierung von Phthalsäureanhydrid aus Phthalid so durchzuführen, daß Ausbeuten an Phthalid über 85 % erhalten werden, auch wenn der Wasserstoffdruck unter 100 bar liegt; weiterhin soll auch technisches Phthalsäureanhydrid als Ausgangsmaterial einsetzbar sein, ohne daß dabei die Ausheute wesentlich zurückgeht.

In Erfüllung dieser Aufgabe wurde nun ein Verfahren zur Herstellung von Phthalid durch katalytische Hydrierung von Phthalsäureanhydrid mit Hilfe eines auf einem Trägermaterial fixierten Nickelkätalysators, dadurch gekennzeichnet, daß die Hydrierung in einem Benzoesäureester mit einer Esterkomponente bis zu 4 Kohlenstoffatomen, und mit einem Nickelkatalysator mit einem Nickelgehalt unter 25 % durchgeführt wird.

Vorzugsweise wird zusätzlich zu dem Renzoesäureester als Lösungsmittel noch ein Alkanol, vorzugsweise Methanol, mitverwendet.

Bei der erfindungsgemäßen Arbeitsweise erhält man Ausbeute an Phthalid, die weit über 75 % liegen. Diese Ausbeuten erhält man auch, wenn man technisches Phthalsäureanhydrid als Ausgangematerial einsetzt, bei dessen Einsatz bei den bekannten Hydrierverfahren auch in Gegenwart von Alkoholen als zusätzliches Lösungsmittel Störungen bei der Hydrierung auftreten.

Überraschenderweise treten diese Ausbeuteverbesserungen nur bei Verwendung von Benzoesäureestern als Lösungsmittel auf. Wenn z.B. die nächsten homologen Verbindungen, z.B. der p-Toluylsäuremethylester, als Lösungsmittel unter sons analogen Bedingungen eingesetzt wird, geht die Ausbeute an Phthalid auf 53 % zurück und die Bildung von o-Toluylsäure als Nebenprodukt steigt auf etwa 28 % an. Nach der US-PS 2,079,325 werden mit Ethylbutyrat und Nickel auf Kieselgur höchstens 70 % Ausbeute erreicht.

Auch der Nickelgehalt des Katalysators spielt eine entscheidende Rolle bei der Ausbeuteverbesserung gegenüber den bekannten Verfahren (s. Vergleichsbeispiele). Der Nikkelgehalt soll deshalb unter 25 % liegen. Dieser Gehalt bezieht sich auf die gesamte Katalysatormasse, d.h. Katalysatorträger plus Nickelverbindung. Dabei werden als Katalysatorträger an sich bekannte Trägermaterialien für Nikkelkatalysatoren eingesetzt, auf denen das metallische Nickel fest fixiert ist. Besonders gute Resultate werden erhalten, wenn der Nickelgehalt des Katalysators zwischen 10 und 15 % liegt; es können jedoch auch Katalysatoren mit einem Nickelgehalt bis herab zu 5 % erfindungsgemäß eingesetzt werden.

Auf dieses Zusammenwirken von Nickelkatalysatoren mit unter 25 % Nickelgehalt und Benzoesäureestern als Lösungsmittel war auch aus der DE-08 2 250 066 kein Hinweis entnehmbar, da dort ein substituiertes Benzochinon zum entsprechenden Hydrochinon mit beliebigen Carbonsäureestern mit u.a. Raney-Nickel ohne Hinweis auf die Wirkung bestimmter Nickelgehalte hydriert wird.

Von den als Lösungsmittel einsetzbaren Benzoesäureslkylestern wird der Benzoesäuremethylester bevorzugt eingesetzt. Es können daher auch die nächst höheren Alkylester eingesetzt werden, deren Esterkomponente bis zu 4 C-Atomen besitzt. Diese Ester werden dann vorzugsweise mit den der Esterkomponente entsprechenden Alkoholen als Lösungsmittel verwendet.

Wenn zusätzlich zu dem Benzoesäureester noch ein Alkanol als Lösungsmittel für die erfindungsgemäße Verfahrensweise dient, dann kann dieser in Mengen bis zu 50 Vol.-%, be zogen auf den Benzoesäureester, anwesend sein. Im allgemeinen liegt der Anteil des Alkanols zwischen 5 und 15 Vol.-%, bezogen auf den Benzoesäureester. Der bevorzugte Alkohol ist Methylalkohol, besonders wenn der Benzoesäure methylester als Lösungsmittel eingesetzt wird.

Der für die erfindungsgemäße Umsetzung notwendige Wasserstoffdruck ist überraschend niedrig. Bei Verwendung von Benzoesäuremethylester mit geringen Anteilen Methanol und den genannten Nickelkatalysatoren ist ein quantitativer Umsatz von Phthalsäureanhydrid mit 10 bis 50, bevorzugt 30 bar Wasserstoffdruck erreichbar. Im allgemeinen genügt ein Wasserstoffdruck bis zu 80 bar für einen quantitative Umsatz des Phthalsäureanhydrids.

Der günstigste Temperaturbereich für die erfindungsgemäße Verfahrensweise liegt zwischen 130 und 180 °C, vorzugsweise zwischen 140 und 160 °C.

Die bei der erfindugnsgemäßen Verfahrensweise in geringer Menge anfallenden sauren Nebenprodukte wie Benzoesäure, Orthotoluylsäure, Phthalsäure und Phthalsäuremonomethylester können durch eine alkalische Behandlung in bekannte Weise entfernt werden. Das auf diese Weise gereinigte Phthalid hat einen Schmelzpunkt von 74 bis 76,5 °C und ei Reinheit, ermittelt gemäß GC-Analyse, von 96 bis 98 %. Phthalid ist ein wertvolles chemisches Zwischenprodukt zu Herstellung von Farbstoffen, Pflanzenschutzmitteln und

2

Pharmazeutika.

## Beispiel 1

Ein 20-Liter-Autoklav mit Hub-Magnet-Rührer wird unter Stickstoff gefüllt mit 5,92 kg Phthalsäureanhydrid (40 Mole), 5 Liter Benzoesäuremethylester, 0,6 kg Methanol und 0,5 kg Nickelkatalysator mit einem Ni-Gehalt von 12 % (Handelsprodukt RCH 12/10 der Farbwerke Hoechst). Nach Aufdrücken auf 30 bar Wasserstoffdruck wird unter Rühren auf 150 °C angebeizt und bei dieser Temperatur im Verlauf von 5 Stunden der Wasserstoffverbrauch im Bereich zwischen 15 und 30 bar durch portionsweise Zugaben ausgeglichen. Nach Einstellen einer Druckkonstanz ließ man noch 15 Minuten nachreagieren. Das erhaltene Gemisch wurde dann auf 100 °C abgekühlt, entspannt und der Katalysator abfiltriert; letzterer wurde noch mit etwa 3 Liter heißem Benzoesäuremethylester nachgewaschen. Das Filtrat wurde eingeengt, zuletzt im Vakuum bis zu einer Sumpftemperatur von 160 °C bei etwa 40 mbar.

Der erhaltene Rückstand vom Gewicht 5,3 kg mit einem Phthalidgehalt von 89,5 Gew.-% wurde zur Entfernung der sauren Nebenprodukte mit 2,6 Liter Methanol und 1,05 kg 25 %igem wässrigem Ammoniak für 15 Minuten zum Rückfluß erhitzt und daraufhin unter Kühlung und Rühren mit 8 Liter Wasser versetzt. Das dabei ausgefallene Phthalid wurde bei Raumtemperatur abfiltiert, mit ca. 1 Liter Wasser nachgewaschen und bei 55 bis 60 °C / 20 mbar getrocknet. Es wurden 4,57 kg Phthalid vom Fp: 74 bis 76,5° (GC 98,3 %) erhalten, entsprechend 85,3 % der Theorie.

## Beispiel 2

Analog Beispiel 1 wurden in einer Laborapparatur 296 g Phthalsäureanhydrid in 250 ml Benzoesäuremethylester unter Verwendung von 40 g des im Beispiel 1 genannten Nickelkatalysators in Gegenwart von 35 g Methanol hydriert. Reaktionsbedingungen und Ausbeuten gehen aus Tabelle 1 hervor.

## Beispiele 3 und 4 (Vergleichsbeispiele)

Analog Beispiel 2 wurden die gleichen Mengen Phthalsaureanhydrid in der gleichen Menge Lösungsmittel hydriert mit dem Unterschied, daß einmal (Beispiel 3) der Nickelgehalt des Katalysators bei 55 % lag und im zweiten Fall (Beispiel 4) anstelle des Benzoesäuremethylesters der p-Toluylsäuremethylester eingesetzt wurde. In beiden Fällen stieg der Anteil an o-Toluylsäure in unerwünschter Weise stark an und auch der Anteil an den weiteren Nebenprodukten wie Phthalsäure und Phtbalsäuremonomethylester war mehr als doppelt so hoch als bei der erfindungsgemäßen Arbeitsweise.

**Tabelle 1**

| Beispiel | 2 | 3 | 4 [1] |
|---|---|---|---|
| Lösungsmittel | BME | BME | PTE |
| Ni-Gehalt des Katalysators (%) | 12 | 55 | 12 |
| Reaktionsbedingungen | | | |
| Druck (bar) | 50 | 50 | 50 |
| Temperatur (°C) | 142 | 148 | 149 |
| Dauer (h) | 5,5 | 2,5 | 6,0 |
| Zusammensetzung des Reaktionsprodukts (%) | | | |
| a) Phthalid | 84,6 | 59,2 | 53,1 |
| b) o-Toluylsäure | 10,5 | 27,5 | 28,3 |
| c) Benzoesäure | 1,7 | 1,4 | – |
| d) Phthalsäure + Phthalsäuremethylester | 2,5 | 6,2 | 5,3 |
| e) sonst. Neben-produkte | 0,7 | 5,7 | 13,3 |

[1] PTE = p-Toluylsäuremethylester

**Patentansprüche**

1. Verfahren zur Herstellung von Phthalid durch katalytische Hydrierung von Phthalsäureanhydrid mit Hilfe eines auf einem Trägermaterial fixierten Nickelkatalysators, dadurch gekennzeichnet, daß die Hydrierung in einem. Benzoesäureester mit einer Esterkomponente bis zu 4 Kohlenstcffatomen, und mit einem Nickelkatalysator mit einem Nickelgehalt unter 25 % durchgeführt wird.

2. Verfahren nach Anspruch 1, dadurch gekennzeichnet, daß die Hydrierung unter Zusatz eines Alkanols, vorzugsweise Methanol, durchgeführt wird.

3. Verfahren gemäß einem der Ansprüche 1 oder 2, dadurch gekennzeichnet, daß die Hydrierung bei Temperaturen zwischen 130 und 180° C, vorzugsweise zwischen 140 und 160° C durchgeführt wird.

4. Verfahren gemäß einem der Ansprüche 1 bis 3, dadurch gekennzeichnet, daß die Hydrierung bei 10 bis 80, vorzugsweise 30 bis 50 bar Wasserstoffdruck durchgeführt wird.

**Claims:**

1. Process for the preparation of phthalide by catalytic hydrogenation of phthalic anhydride with the assistance of a nickel catalyst fixed on a support material, characterised in that the hydrogenation is carried out in a benzoic acid ester with an ester component of up to 4 carbon atoms and with a nickel catalyst with a nickel content below 25%.

2. Process according to claim I, characterised in that the hydrogenation is carried out with the addition of an alcohol, preferably methanol.

3. Process according to one of claims 1 or 2, characterised in that the hydrogenation is carried out at temperatures between 130 and 180°C, preferably between 140 and 160°C.

4. Process according to claims 1 to 3, characterised in that the hydrogenation is carried out at 10 to 80, preferably 30 to 50 bar hydrogen pressure.

**Revendications**

1. Procédé de préparation du phtalide par hydrogénation catalytique de l'anhydride phtalique à l'aide d'un catalyseur au nickel fixé sur une matière de support, procédé caractérisé en ce qu'on effectue l'hydrogénation dans un ester de l'acide benzoïque comportant un composant ester ayant jusqu'à 4 atomes de carbone, et avec un catalyseur au nickel ayant une teneur en nickel inférieure à 25 %.

2. Procédé selon la revendication 1, caractérisé en ce qu'on effectue l'hydrogénation en ajoutant un alcanol, avantageusement le méthanol.

3. Procédé selon l'une des revendications 1 ou 2, caractérisé en ce qu'on effectue l'hydrogénation à des températures comprises entre 130 et 180° C, avantageusement entre 140 et 160°C.

4. Procédé selon l'une des revendications 1 à 3, caractérisé en ce qu'on effectue l'hydrogénation sous une pression d'hydrogène de 10 à 80, avantageusement de 30 à 50 bars.